# EUROPEAN PATENT SPECIFICATION

(11) **EP 0 589 991 B1**
(45) Date of publication and mention of the grant of the patent: **27.08.1997**
(21) Application number: 92912523.5
(22) Date of filing: 19.06.1992
(51) Int. Cl.: G01N 33/52

(54) **Test member for assessing a component in a bodily fluid**
Testvorrichtung zur Bestimmung von einer in einer körperlichen Flüssigkeit enthaltenen Verbindung
Dispositif de test pour détecter un composant dans un fluide corporel

(30) Priority: 19.06.1991 GB 9113213
(43) Date of publication of application: 06.04.1994
(73) Proprietor: HYPOGUARD (UK) LIMITED, Woodbridge Suffolk IP12 1PE (GB)
(72) Inventor: COX, Stephen, John, Felixstowe Suffolk IP11 9PJ (GB); GULLICK, Stephen, Peter, Ipswich Suffolk IP5 7PQ (GB)
(74) Representative: Dummett, Thomas Ian Peter
(86) International application number: GB9201117
(87) International publication number: WO9222814

(56) References cited:
- EP-A- 0 256 806
- EP-A- 0 265 253
- GB-A- 1 311 324
- US-A- 3 443 903
- US-A- 4 328 184
- Concise Oxford Dictionary, 7th Edition, page 272

## Description

The present invention relates to a test member for assessing a component in a fluid to be tested, and to a method for testing a fluid which comprises applying it to a membrane of said test member.

### BACKGROUND TO THE INVENTION:

Samples of blood or other bodily fluids such as urine are often tested to assess the level of some component therein, such as blood sugars, cholesterol or urea in the sample. Typically, this is done by applying a droplet of the fluid to a support medium which carries the appropriate test reagents thereon or therein. The support medium is usually a strip of white plastic or the like with a pad of the reagents applied to one side adjacent the end thereof. In the case of a blood test stick, the reagent pad usually comprises a gelatin or other matrix containing a mixture of the enzymes glucose oxidase and peroxidase and the chromogen o-tolidine. The chromogen is coloured blue when glucose in the blood is oxidised to gluconic acid and hydrogen peroxide and the hydrogen peroxide reacts with the o-tolidine.

Such test sticks suffer from a number of problems in that the blood sample is exposed to the environment at all times both during and after the test. As a result, there is a risk of contamination of the sample and of cross-contamination via the operator from one sample to the next. Problems also arise in disposing of the blood-carrying test stick at the end of the test. Furthermore, the presence of excess red blood cells often affects the colour detected and hence the perceived level of the material being tested for.

In order to reduce these problems, it has been proposed to apply the reagent pad to one face of a porous membrane and to apply the blood sample to the other face of the membrane so that it penetrates through the membrane to react with the reagent pad. The membrane is then observed from the side carrying the reagent pad using a conventional strip scanning technique. Whilst such a proposal suffers from the potential of cross-contamination, the risks are reduced because the scanned face of the membrane faces inwardly in the scanning device and hence the blood sample does not contact the device itself. Furthermore, it is not necessary to remove excess blood as with a conventional blood test stick because the majority of any excess blood remains on the face of the membrane which is not being scanned and hence has a reduced effect on the colour which is observed at the other face of the membrane.

For convenience, test methods in which the colour development is observed from the opposite face of a carrier member to that face of the member to which the blood or other sample to be assessed has been applied will be denoted herein as back read test methods.

We have devised a modification to the back read test method in which the sample is held within a device having a chamber which has a porous membrane as one wall thereof. The membrane carries or is impregnated with the matrix/enzyme/chromogen mixture so that a colour is developed in the mixture when blood is applied to one face of the membrane. This colour is then observed from the opposed face of the membrane. In this way, the blood sample is held within the chamber and is not exposed to the environment and there is reduced risk of the operator coming into contact with the blood. Disposal is also assisted in that the device can be disposed of without the blood escaping from the chamber.

Back reading techniques find especial use where the sample contains cellular material and it is desired to separate this from a plasma component containing no cellular material for the test. The membrane carrying the reagent mixture can be structured so that the cellular material is held upon the face of the membrane to which the sample is applied and the non-cellular or plasma fluid penetrates the membrane via the pores to develop the colour which is observed from the other face of the membrane. In order to minimise cell rupture and hence colour contamination of the reagent mixture, it is preferred to blind the pores of the membrane as described in PCT Application No GB92/01118 (WO-A-92/22 815).

The back read test method also offers the advantage that the colour in only a small area of the membrane needs to be scanned and this should in theory enable a smaller blood sample to be used, further reducing the problems of contamination and disposal. However, other problems are encountered in achieving satisfactory results with small blood samples.

The membrane can be cut into a disc or small pad of material which is then mounted on a translucent support strip, typically by laminating the disc or pad between two sheets of the support material. The strip is then mounted in the scanning device with the disc or pad aligned with the scanning element so that the device observes the colour developed in the pad or disc. In order to assist alignment and to reduce extraneous optical interference, the disc or pad is often surrounded by an opaque annulus or other surround.

In theory, the sample reacts with the reagents in the membrane in the disc or pad in the immediate vicinity to which the sample is applied. This should result in colour being developed in the area to be scanned and hence avoids the need to apply sufficient blood to colour the whole of the reagent area of the test strip, as is required with conventional techniques.

However, we have found that this is not achieved in practice and that, whilst the initial colour develops in the desired position, it bleeds away. The sample must therefore be observed within a very strictly defined time span if consistent results are to be achieved. We have found that this bleeding away of the colour is due to the sample diffusing away from the disc or pad.

We have found that the problem of bleeding of the colour can be reduced if the disc or pad is surrounded by a closed air gap which provides a circumferential flow barrier so that the sample is retained within the disc or pad. As a result, more consistent results can be achieved and the amount of sample required can be consistently less than that required when a conventional test strip is used or when the hitherto proposed back read techniques are used.

### SUMMARY OF THE INVENTION:

Accordingly, the present invention provides a test member for assessing a component in a fluid to be tested, characterised in that the test member comprises a support member carrying a membrane impregnated with or carrying one or more reagents required for the test, which membrane is surrounded at least circumferentially by a flow barrier which inhibits flow of the sample fluid through the lateral edges of the membrane, the flow barrier being provided by a closed air gap around the circumferential periphery of the membrane which is sufficient to break the capillary flow from the edge of the membrane.

Preferably, the membrane is made from a micro-porous material which is impregnated with a gel matrix containing a mixture of one or more enzyme reagents with one or more chromogen materials, which mixture is adapted to interact with at least one of the non-cellular component of a fluid sample applied to one face of the membrane so as to develop a colour in the visible spectrum which can be observed from the opposed face of the membrane. Preferably, the gel matrix blinds a substantial length of at least a major proportion of the pores in the membrane so that rupture of cell walls in cellular components of the material to be tested is reduced.

Preferably, the support member is a white or opaque plastics strip having an aperture therethrough in register with the membrane.

The support member and the membrane can take any suitable form, depending upon the scanning technique which is to be used. Thus, the invention can be applied to a conventional axially elongated blood test stick in which the support member is the stick. Such a stick is usually formed from an opaque white plastic carrying the membrane at or adjacent one end thereof. In this case, the sample is applied to the exposed upper face of the membrane and the colour which develops in the membrane is observed after a specified time from the exposed upper face of the membrane and the back read technique is not used. The invention, in this case, reduces lateral spread of the colour away from a small area of the reagent mixture in the membrane and thus enables a smaller sample to be used than has been hitherto required in a conventional front read test strip.

However, the invention is of especial benefit in test methods using the back read test method. It is therefore preferred that the support member either be translucent so that the colour developed in the membrane can be observed through the strip; or that the strip be formed with an aperture therethrough in register with the membrane so that the colour can be observed directly through that aperture. Again the plan shape of the membrane and support member can be of any suitable shape or form to operate in the scanning device for which the test member is intended, for example square, rectangular, polygonal or, preferably, circular.

The membrane is preferably sized so that it becomes saturated with the fluid sample applied to it so that the fluid is substantially uniformly distributed throughout the membrane.

The support member is typically self supporting and acts as the sole support for the membrane. However, it is within the scope of the invention to apply the membrane to an intermediate support which is then held in a rigid member. For example, the membrane can be provided by a thin micro-porous membrane which is held by a suitable ring clamp or other means to form the end face of an open ended chamber in a metal or plastic capsule or other receptacle into which a sample of blood is introduced so that the sample contacts the internal face of the membrane.

For convenience, the invention will be described hereinafter in terms of a disc or pad of micro-porous membrane which is secured to one face of a disc or rectangle of a polyvinyl or other sheet plastic as the self supporting support member.

The support member, the membrane for the disc or pad and the reagent mixture applied to or impregnated into the membrane may be selected from a wide range according to the type of test which is to be carried out. As stated above, the back read technique is of especial application in testing the non-cellular component of a bodily or other fluid which contains one or more cellular components which are separated from the fluid under test by the micro-porous nature of the membrane. The invention can thus be used in monitoring plant fluids as well as bodily or other fluids.

For convenience, the invention will be described hereinafter in terms of testing for glucose in a blood sample.

The membrane will typically have a mixture of a gel matrix, one or more enzymes and one or more chromogens applied as a coating to one face thereof. Preferably, the membrane is impregnated with the mixture so that the mixture is substantially uniformly distributed throughout the membrane and preferably blinds substantially all of the pores in the membrane for at least 50%, preferably at least 75%, of their length. The nature of the mixture and the amount loaded onto the membrane can readily be selected using criteria known in the art.

The membrane is surrounded by a flow barrier. Where the flow barrier is provided by a barrier coating or collar which surrounds the periphery of the membrane, gaps or cracks may be formed between the barrier coating or member and the support member so that capillary flow takes place from the edge of the membrane disc through those cracks or gaps. The better the manufacturing technique, the smaller those cracks or gaps will be, but the surface tension effects drawing fluid into them will increase as the cross-sectional dimensions of any such gaps or cracks decreases.

Surprisingly, we have found that if a closed air gap is deliberately formed around the circumferential periphery of the membrane this can be proportioned so that the surface tension properties of the fluid under test are insufficient to bridge that gap and hence that capillary flow across the gap does not take place to any significant extent. In this way, the disc or pad of the reagent carrying membrane can be formed as small as is compatible with the scanning technique used so that a small amount of sample is required, yet the colour from the small sample is retained in the area to be scanned and does not significantly bleed away laterally.

Accordingly, from a preferred embodiment, the present invention provides a reagent carrying membrane supported on a support member, to which membrane a fluid sample is to be applied to generate a coloured component by interaction with the reagent, which colour is to be observed over a given area of the membrane, characterised in that the circumference of the membrane is surrounded by a closed air gap sufficient to prevent capillary flow of the coloured component radially outward from the edge of the membrane. Preferably, the membrane is sufficiently small radially that a sample of fluid applied to one face of the membrane does not diffuse radially beyond the area over which the coloured component is to be observed within the time period of the observation. Preferably, the membrane is sized so that it becomes saturated with the fluid applied to it.

The air gap can be formed in a number of ways, for example by locating the disc of the membrane within a larger recess in a support member which is made from or is coated with a material which is not significantly wet by the fluid applied to the membrane. However, a particularly preferred method is to apply the membrane to the support member and to secure the membrane is place by an annular ring of a non-wetted material which extends radially beyond the edge of the membrane disc. Pressure is not applied to the annular ring in the region of the circumferential edge of the membrane disc so that the ring does not conform to the step between the upper face of the membrane disc and the adjacent face of the support member. Thus, for example, the annular ring can be secured to the upper face of the membrane and to the support member around but radially displaced from the edge of the membrane disc by ultrasonic or heat welding, adhesive or other techniques, so that the ring forms a triangular air gap between the edge of the membrane, the face of the support member and the underside of the ring.

The optimum size and shape for the air gap can readily be determined by simple trial and error test and from a knowledge of the rheological properties of the fluid under test and the surface energies of the materials of the membrane, the support member and the securing ring.

Typically, the annular securing ring will be made from a silicone rubber sheet, a sheet or woven polyamide, or other material which is not readily wetted by the material to be tested, and will be formed with a circular aperture therein which is to register with the area over which the colour is to be observed.

### DESCRIPTION OF THE DRAWINGS:

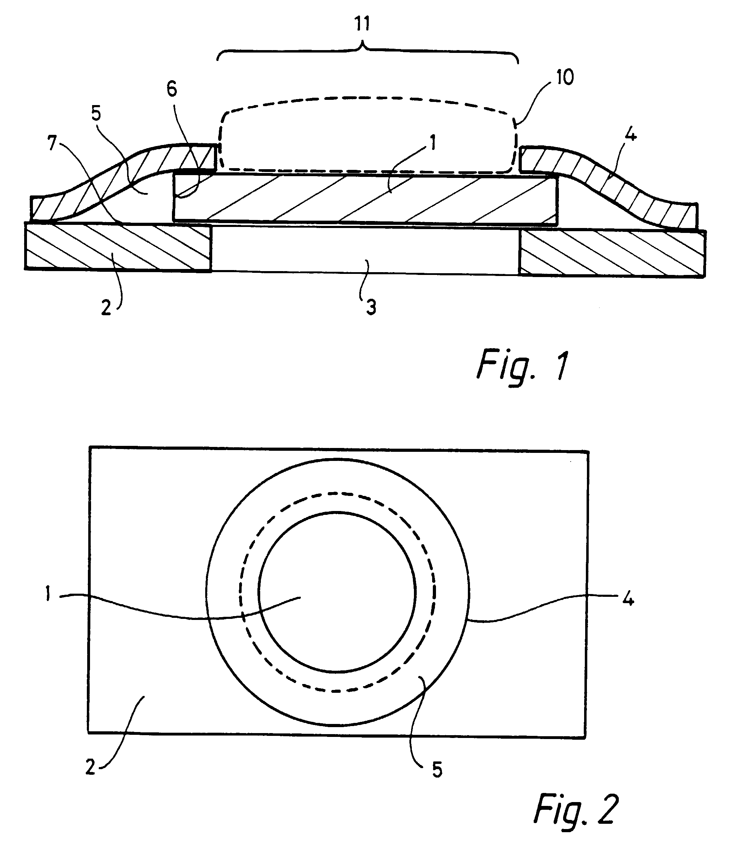

The membrane of the invention finds especial use in the back read technique for testing for glucose and other materials in blood or other bodily fluids and a preferred form thereof for such use will now be described by way of illustration with respect to the accompanying drawings in which Figure 1 is a vertical cross-section through a membrane and its support; and Figure 2 is a plan view from above of the membrane of Figure 1.

### DESCRIPTION OF THE PREFERRED EMBODIMENT:

A membrane disc 1, 6 mm in diameter, was cut from a sheet of a commercially available modified polysulfone micro-porous resin sheet 0.2 to 0.4 mm thick, which had an average pore diameter of 0.2 micrometres and had an air permeablilty of 3 litres per minute per square centimetre at an applied pressure of 69 kPa (10 psig).

The sheet had been impregnated with an aqueous mixture of glucose oxidase, peroxidase and o-tolidine in a gelatin matrix to provide 5 IU of glucose oxidase, 3 IU of peroxidase, 0.2 milligrams of o-tolidine and 4 milligrams of gelatin per square cm of the membrane.

The membrane disc was applied to a white PVC plastic support rectangle 2, 18 mm wide by 24 mm long, with an aperture 3, 5.2 mm diameter through it which registered with the membrane disc 1.

An annular ring 4 of a polyamide fabric was secured by adhesive to the exposed face of the membrane disc 1 and to the rectangle 2 so that the ring 4 formed a closed triangular air gap 5 between the edge 6 of the disc and the upper face 7 of the surrounding area of the support rectangle 2. This air gap substantially eliminated the surface tension bridge between the edge of the disc and its surroundings. The ring 4 was secured so that its annulus was substantially coaxial with the aperture 3 in the support rectangle 2 so that the disc 1, the ring 4 and the aperture 3 were radially substantially symmetrically aligned with oneanother and the air gap 5 was substantially uniform around the circumference of disc 1.

When a droplet of blood 10 is applied to the upper face of the membrane 1 through the central aperture 11 in ring 4, the blood plasma penetrates into the pores of the membrane and interacts with the enzymes and chromogen in the gelatin matrix in the pores to produce a blue colouration. This colouration can be observed through the aperture 3 in the support 2 to give an indication of the glucose level in the blood sample. Due to the air gap 5, the blood plasma cannot all bleed radially outward from the membrane, so that the colouration is retained in the area of the membrane to be observed. By sizing the membrane so that the area observed corresponds substantially to the area underlying the central aperture 11 in ring 4 and by sizing ring 4 so that it overlies only the peripheral edge of the membrane disc 1, the loss of colouration by diffusion to the peripheral area of the membrane is reduced and the effect of that diffusion is minimised. As a result, only a small sample of blood need be used and substantially consistent results can be achieved with that small size of sample.

By way of comparison, when steps are taken to avoid the formation of air gap 5, the colouration migrates beyond the edge of the membrane disc and the colouration observed through aperture 3 varies with time and with the precise portion of the disc which is observed.

## Claims

1. A test member for assessing a component in a fluid to be tested, characterised in that the test member comprises a support member (2) carrying a membrane (1) impregnated with or carrying one or more reagents required for the test, which membrane (1) is surrounded at least circumferentially by a flow barrier (5) which inhibits flow of the sample fluid through the lateral edges of the membrane (1), the flow barrier (5) being provided by a closed air gap (5) around the circumferential periphery of the membrane (1) which is sufficient to break the capillary flow from the edge of the membrane (1).

2. A test member as claimed in claim 1, characterised in that the membrane (1) is made from a micro-porous material which is impregnated with a gel matrix containing a mixture of one or more enzyme reagents with one or more chromogen materials, which mixture is adapted to interact with one or more non-cellular components of a fluid sample (10) applied to one face of the membrane (1) so as to develop a colour in the visible spectrum which can be observed from the opposed face of the membrane (1).

3. A test member as claimed in either of claims 1 or 2, characterised in that the reagent mixture is in the form of a gel matrix which blinds a substantial length of at least a major proportion of the pores in the membrane (1) so that rupture of cell walls in cellular components of the material (10) to be tested is reduced.

4. A test member as claimed in any one of the preceding claims, characterised in that the support member (2) is an axially elongated member carrying the membrane (1) adjacent one end of the elongated member.

5. A test member as claimed in any one of the preceding claims, characterised in that the support member (2) is either translucent and/or has an aperture therethrough in register with the membrane (1) so that the colour developed in the membrane (1) can be observed through the support member (2).

6. A test member as claimed in any one of the preceding claims, characterised in that the membrane (1) is in the form of a disc which forms the end face of an open ended chamber (11) in a sample receiving member (4) into which a sample of the fluid (10) to be tested is to be introduced so as to contact one face of the membrane (1).

7. A test member comprising a reagent-carrying membrane (1) supported on a support member (2), to which membrane (1) a fluid sample (10) is to be applied to generate a coloured component by interaction with the reagent, which colour is to be observed over a given area of the membrane, characterised in that the circumferential periphery of the membrane is surrounded by a closed air gap (5) sufficient to prevent capillary flow of the coloured component radially outward from the edge of the membrane (1).

8. A method for testing a fluid (10) which comprises applying it to a membrane (1) of a test member as claimed in any one of the preceding claims.

9. A method as claimed in claim 8, characterised in that the fluid (10) is blood which is applied to one face of the membrane (1), the membrane (1) carries or is impregnated with a reagent mixture which develops a colour upon reaction with one or more components of the blood, and the colour is observed at the opposite face of the membrane to that to which the sample is applied.

## Patentansprüche

1. Testvorrichtung zur Bestimmung einer Komponente in einem zu testenden Fluid,
dadurch **gekennzeichnet**,
daß die Testvorrichtung einen Träger (2) aufweist, der eine Membran (1) trägt, die mit einem für den Test erforderlichen Reagenz oder mit mehreren Reagenzien getränkt ist oder diese trägt, daß die Membran (1) zumindest am Umfang durch eine Flußbarriere (5) umgeben ist, welche den Fluß des Probenfluids durch die seitlichen Ränder der Membran (1) verhindert und daß die Flußbarriere (5) durch einen zur Unterbrechung des Kapellarflusses von dem Rand der Membran (1) ausreichenden, geschlossenen Luftraum (5) um die Umfangsperipherie der Membran (1) gebildet ist.

2. Testvorrichtung nach Anspruch 1,
dadurch **gekennzeichnet**,
daß die Membran (1) aus einem mikroporösen Material aufgebaut ist, welches mit einer Gelmatrix getränkt ist, die ein Gemisch aus einem oder mehreren Enzymreagenzien mit einem oder mehreren Chromogenmaterialien enthält und daß das Gemisch mit einer oder mehreren nicht-cellulären Komponenten einer auf eine Seite der Membran (1) aufgebrachten Fluidprobe (10) interagieren kann, um eine Farbe in dem sichtbaren Spektrum zu entwickeln, die von der gegenüberliegenden Seite der Membran (1) beobachtet werden kann.

3. Testvorrichtung nach Anspruch 1 oder 2,
dadurch **gekennzeichnet**,
daß das Reagenzgemisch in Form einer Gelmatrix vorliegt, eine beträchtliche Länge mindestens eines Hauptbereiches der Poren in der Membran zusetzt, so daß ein Brechen der Zellwände in den cellulären Komponenten des zu testenden Materials (10) reduziert wird.

4. Testvorrichtung nach einem der vorhergehenden Ansprüche,
dadurch **gekennzeichnet**,
daß der Träger (2) ein in axialer Richtung längliches Teil darstellt, welches die Membran (1) in der Nähe eines Endes des länglichen Teiles trägt.

5. Testvorrichtung nach einem der vorhergehenden Ansprüche,
dadurch **gekennzeichnet**,
daß der Träger (2) entweder transluzent ist und/oder eine Öffnung dort hindurch in Ausrichtung mit der Membran (1) besitzt, so daß die in der Membran (1) entwickelte Farbe durch den Träger (2) beobachtet werden kann.

6. Testvorrichtung nach einem der vorhergehenden Ansprüche,
dadurch **gekennzeichnet**,
daß die Membran (1) die Form einer Scheibe besitzt, welche die Endseite einer Kammer (11) mit einem offenen Ende in einem Probeaufnahmeelement (4) bildet, in welches eine Probe des zu testenden Fluids (10) eingeführt wird, so daß eine Seite der Membran (1) in Kontakt damit kommt.

7. Testvorrichtung mit einer durch einen Träger (2) gestützten, ein Reagenz tragende Membran (1), auf die eine Fluidprobe (10) zur Erzeugung einer gefärbten Komponente durch Interaktion mit dem Reagenz aufgetragen wird, wobei die Farbe über eine gegebene Zone der Membran beobachtet wird,
dadurch **gekennzeichnet**,
daß die Umfangsperipherie der Membran durch einen zur Verhinderung eines kapellaren Flusses der gefärbten Komponente radial auswärts von der Seite der Membran (1) ausreichenden, geschlossenen Luftraum (5) umgeben ist.

8. Verfahren zum Testen eines Fluids (10), bei dem dieses auf eine Membran (1) einer Testvorrichtung nach einem der vorhergehenden Ansprüche aufgebracht wird.

9. Verfahren nach Anspruch 8,
dadurch **gekennzeichnet**,
daß es sich bei dem Fluid (10) um Blut handelt, das auf eine Seite der Membran (1) aufgebracht wird, wobei die Membran (1) ein Reagenzgemisch trägt oder damit getränkt ist, welches nach Reaktion mit einem oder mehreren Blutbestandteilen eine Farbe entwickelt, und daß die Farbe auf der Seite der Membran, die derjenigen gegenüberliegt, auf der die Probe aufgetragen wird, beobachtet wird.

## Revendications

1. Dispositif d'analyse pour détecter un composant dans un fluide devant être analysé, caractérisé par le fait que le dispositif d'analyse comprend un élément support (2) supportant une membrane (1) imprégnée par ou supportant un ou plusieurs réactifs nécessaires pour l'analyse, laquelle membrane (1) est entourée au moins sur sa circonférence par une barrière d'écoulement (5) qui empêche l'écoulement du fluide d'échantillon par les bords latéraux de la membrane (1), la barrière d'écoulement (5) étant dotée d'un espace d'air fermé (5) autour de la circonférence de la membrane (1) qui est suffisant pour rompre l'écoulement capillaire à partir du bord de la membrane (1).

2. Dispositif d'analyse selon la revendication 1, caractérisé par le fait que la membrane (1) est faite à partir d'un matériau microporeux qui est imprégné par une matrice de gel contenant un mélange d'un ou de plusieurs réactifs enzymatiques avec une ou plusieurs matières chromogènes, lequel mélange est apte à interagir avec un ou plusieurs composants non cellulaires d'un échantillon fluide (10) appliqué sur une face de la membrane (1) de façon à développer une couleur dans le spectre visible qui peut être observée à partir de la face opposée de la membrane (1).

3. Dispositif d'analyse selon l'une des revendications 1 ou 2, caractérisé par le fait que le mélange réactif est sous la forme d'une matrice de gel qui obstrue une longueur importante d'au moins une proportion majeure des pores dans la membrane (1) de façon à ce que la rupture des parois de cellules dans les composants cellulaires de la matière (10) devant être analysée soit réduite.

4. Dispositif d'analyse selon l'une quelconque des revendications précédentes, caractérisé par le fait que l'élément support (2) est un élément allongé axialement supportant la membrane (1) adjacente à une extrémité de l'élément allongé.

5. Dispositif d'analyse selon l'une quelconque des revendications précédentes, caractérisé par le fait que l'élément support (2) est ou translucide et/ou présente une ouverture traversante coïncidant avec la membrane (1) de façon à ce que la couleur développée dans la membrane (1) puisse être observée à travers l'élément support (2)

6. Dispositif d'analyse selon l'une quelconque des revendications précédentes, caractérisé par le fait que la membrane (1) est sous la forme d'un disque qui forme la face d'extrémité d'une chambre (11) à extrémité ouverte dans un élément (4) recevant un échantillon dans lequel un échantillon du fluide (10) devant être analysé doit être introduit de façon à être en contact avec une face de la membrane (1).

7. Dispositif d'analyse comprenant une membrane (1) supportant un réactif, supportée par un élément support (2), un échantillon fluide devant être appliqué sur la membrane (1) pour générer un composant coloré par interaction avec le réactif, laquelle couleur devant être observée sur une surface donnée de la membrane, caractérisé par le fait que la circonférence de la membrane est entourée par un espace d'air fermé (5) suffisant pour empêcher l'écoulement capillaire du composant coloré radialement vers l'extérieur à partir du bord de la membrane (1).

8. Procédé d'analyse d'un fluide (10) qui comprend l'application à une membrane (1) d'un dispositif d'analyse tel que revendiqué à l'une quelconque des revendications précédentes.

9. Procédé selon la revendication 8, caractérisé par le fait que le fluide (10) est du sang qui est appliqué sur une face de la membrane (1), la membrane porte ou est imprégnée par un mélange réactif qui développe une couleur lors de la réaction avec un ou plusieurs composants du sang, et la couleur est observée à la face de la membrane opposée à celle sur laquelle est appliqué l'échantillon.
